# EUROPÄISCHE PATENTSCHRIFT

(11) **EP 0 651 265 B1**
(45) Veröffentlichungstag und Bekanntmachung des Hinweises auf die Patenterteilung: **28.03.2001**
(21) Anmeldenummer: 94114933.8
(22) Anmeldetag: 22.09.1994
(51) Int. Cl.: G02B 5/22, A61F 9/02, G02C 7/10

(54) **Laserschutzbrille**
Laser safety glasses
Lunette de protection laser

(30) Priorität: 27.10.1993 DE 4336586
(43) Veröffentlichungstag der Anmeldung: 03.05.1995
(73) Patentinhaber: Carl Zeiss, 89518 Heidenheim (Brenz) (DE); CARL ZEISS-STIFTUNG HANDELND ALS CARL ZEISS, D-89518 Heidenheim (Brenz) (DE)
(72) Erfinder: Schürle, Hermann, D-73430 Aalen (DE)

(56) Entgegenhaltungen:
- EP-A- 0 356 890
- WO-A-85/00391
- DD-A- 286 569
- ASTRONOMY AND ASTROPHYSICS, Bd. 276, Nr. 2, September 1993 GERMANY, Seiten 655-662, F.CHOLLET ET AL.
- SOVIET JOURNAL OF OPTICAL TECHNOLOGY, Bd. 57, Nr. 1, Januar 1990 USA, Seiten 37-39, O.DYMSHITS ET AL.

## Beschreibung

Die Erfindung betrifft eine Laserschutzbrille mit einem Laserschutzfilter gegen Strahlung von CO₂-Lasern (Wellenlänge 10.600 nm), welches aus einer oder mehreren Schichten aufgebaut ist, wobei mindestens eine der Schichten aus dem Material "Zerodur" (Warenzeichen der Firma Glaswerke Schott) hergestellt ist.

Laserschutzfilter für CO₂-Laser sind bekannt. So ist ein Laserschutzfilter bekannt, welches aus dem Material "Duran" (Warenzeichen der Firma Glaswerke Schott) besteht. Dieses Material hat einen linearen Wärmeausdehnungskoeffizienten im Bereich 20-300° C von größer als 3,25 x 10⁻⁶/°C. Duran selber ist ein technisches Glas Borosilikatglas mit niedrigem Wärmeausdehnungskoeffizienten und hoher Temperaturwechselbeständigkeit.

Aus Astronomy and Astrophysics, Bd. 276 (1993), S. 655-662, ist der Einsatz eines Filters, welches aus mehreren Schichten aufgebaut ist, und wobei eine der Schichten aus dem Material Glaskeramik "Zerodur" hergestellt ist, bekannt. Das Filter enthält eine transparente Zerodur-Schicht, die mit einer Chrom-Nickel Beschichtung versehen ist, und wird als Filter für die Sonnenbeobachtung eingesetzt.

Es ist die Aufgabe der Erfindung den Einsatzbereich von Laserschutzfiltern, die mindestens eine Schicht aus Zerodur aufweisen, zu vergrößern.

Diese Aufgabe wird erfindungsgemäß dadurch erfüllt, daß ein Laserschutzfilter, bei dem mindestens eine Schicht aus dem Material "Zerodur" hergestellt ist, in Schutzbrillen eingesetzt wird.

Die Transmission für CO₂-Laserstrahlung ist für Zerodur kleiner als 10⁻⁶. Eine Prüfung speziell für Zerodur ergab eine Leistungsdichte für CO₂-Laser von 10⁹W/m² nach DIN 58215, was einer Schutzstufe von mindestens L6A entspricht. Bisher ist kein CO₂-Laserschutzfilter mit einer derart hohen Schutzstufe bekannt.

Der Einsatz solcher Schutzfilter als Kabinenfenster ist ohne weiteres möglich.

Das Schutzfilter sollte dabei als Sandwich-Filter aufgebaut sein. Wichtig ist auch, daß der Wärmeausdehnungskoeffizient des Filters möglichst gering ist. Die Transmission des Filters sollte im spektralen Bereich genügend groß sein und die Brechzahl und Abbe-Zahl in einem Bereich liegen, welche es erlaubt, eine Schicht des Sandwich-Filters als Korrektionsglas auszuführen.

Die Erfindung wird im folgenden anhand der Figuren näher erläutert, wobei weitere wesentliche Erfindungsmerkmale beschrieben werden.

Dabei zeigen
- Fig. 1: ein erstes in einer Laserschutzbrille eingesetztes erfindungsgemäßes Laserschutzfilter; und
- Fig. 2: ein zweites in einer Laserschutzbrille eingesteztes erfindungsgemäßes Laserschutzfilter.

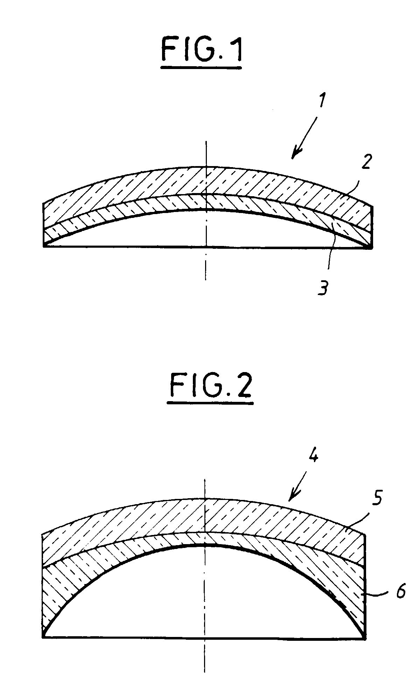

Das in Fig. 1 dargestellte Laserschutzfilter (1) ist ein Planfilter und besteht aus zwei miteinander verklebten Planscheiben (2, 3). Das Material beider Scheiben (2, 3) ist die Glaskeramik Zerodur. Glaskeramik als solche wird in vielen Anwendungsgebieten schon als Werkstoff für optische Teile verwendet (allerdings nur als Spiegelträgermaterial).

Glaskeramik ist ein anorganischer, porenfreier Werkstoff, der eine kristalline Phase und eine Restglasphase enthält. Dies sorgt für eine geringe thermische Längenausdehnung. Die in der Regel sehr kleinen Kristalle und der geringe Brechzahlunterschied zwischen Kristall- und Gasphase ergeben eine sehr gute Transparenz im Spektralbereich von ca. 0,4 - 2,3 µm. Glaskeramik hat eine völlig richtungsfreie Struktur und läßt sich mit den gleichen Maschinen und Werkzeugen wie optische und technische Gläser bearbeiten.

Die beiden miteinander verklebten Planscheiben (2, 3) sollten zusammen eine Dicke von nicht weniger als 4mm besitzen. Dabei beträgt die Schichtdicke der vorderen Planscheibe (2) 2,5 mm und die Schichtdicke der hinteren Planscheibe (3) beträgt 1,5 mm. Aber auch größere Dicken sind je nach der Leistung des CO₂-Lasers sinnvoll (z.B. 6,5 mm). Dabei sollte man möglichst die Dicke des Laserschutzfilters (1) der Leistung des Lasers anpassen, damit die Laserschutzbrille (in den Fig. nicht dargestellt) mit dem oder den Laserschutzfiltern (1) nicht zu schwer wird.

Anderseits kann das Laserschutzfilter auch als Planplatte aus Glaskeramik hergestellt sein, um eine Kabine oder einen sonstigen Arbeitsplatz vor Laserstrahlen zu sichern.

In der Fig. 2 ist nun dargestellt, daß das Laserschutzfilter (4) auch als Korrektionsfilter realisiert werden kann. Dabei sind auch hier zwei Scheiben (5, 6) aus der Glaskeramik Zerodur miteinander verklebt. Die äußere Scheibe (5) dient als Filter und die innere Scheibe (6) als Korrektionsglas. Da die Feinbearbeitung (Schleifen und Polieren) herkömmlichen optischen Gläsern nicht nachsteht und auch die optischen Eigenschaften insbesondere von Zerodur (Brechzahl n_{d} = 1.542 und Abbe-Zahl γ _{d} = 58,8) entsprechend sind, haben Linsen aus Zerodur eine entsprechende optische Qualität wie Glaslinsen.

Selbstverständlich müssen nicht alle Scheiben (5, 6) des Laserschutzfilters (4) aus Glaskeramik hergestellt sein. Insbesondere die rückseitige Scheibe (6) kann aus jedem beliebigen Material bestehen. Das Laserschutzfilter braucht auch nicht nur aus zwei Scheiben bestehen, sondern kann aus einer beliebigen anzahl von Scheiben aufgebaut sein. Allerdings wird man die Anzahl der Scheiben möglichst gering halten, da jede zusätzliche Scheibe zwei zusätzliche, optisch zu bearbeitende Flächen benötigt.

Generell kann das Filtert jede beliebige Oberflächenform besitzen, insbesondere kann das Laserschutzfilter als Planplatte angefertigt sein, wobei die Dicke des Filters in Abhängigkeit zur Laserleistung und zur Standzeit (Wider-) des Filters auszuwählen ist.

## Patentansprüche

1. Laserschutzbrille in welche ein Laserschutzfilter (1,4) gegen Strahlung von CO₂-Lasern eingesetzt ist, welches aus einer oder mehreren Schichten (2,3,5,6) aufgebaut ist, wobei mindestens eine der Schichten aus dem Material Glaskeramik "Zerodur" (Warenzeichen der Firma Glaswerke Schott) hergestellt ist.

2. Laserschutzbrille nach Anspruch 1, wobei die aus "Zerodur" bestehende Schicht des Filters (4) als äußere Scheibe (5) vor den folgenden Schichten angeordnet ist.

3. Laserschutzbrille nach einem der Ansprüche 1-2, dadurch gekennzeichnet, daß der lineare Wärmeausdehnungskoeffizient im Bereich 20°C -300°C des Filters (1, 4) kleiner als 3 x 10⁻⁶/° C (beim Raumtemperatur) ist.

4. Laserschutzbrille nach einem der Ansprüche 1-3, dadurch gekennzeichnet, daß der lineare Wärmeausdehnungskoeffizient im Bereich 20°C - 300°C des Filters (1, 4) kleiner als 0,1 x 10⁻⁶/° C ist.

5. Laserschutzbrille nach einem der Ansprüche 1-4, dadurch gekennzeichnet, daß das Filter (1, 4) aus zwei Glaskeramik-Schichten (2, 3, 5, 6) aufgebaut ist.

6. Laserschutzbrille nach einem der Ansprüche 1-5, dadurch gekennzeichnet, daß die innere, hintere Schicht (6) des Filters (4) als Korrektionsglas ausgeführt ist.

## Claims

1. Laser protective goggles into which a laser protective filter (1, 4) against radiation from CO₂ lasers is inserted, which filter is constructed from one or more layers (2, 3, 5, 6), at least one of the layers being produced from the glass ceramic material of "Zerodur" glass ceramic (trademark of the company Glaswerke Schott".

2. Laser protective goggles according to Claim 1, in which the layer, consisting of "Zerodur", of the filter (4) is arranged as an outer coating (5) in front of the following layers.

3. Laser protective goggles according to one of Claims 1-2, characterized in that, in the range of 20°C - 300°C, the linear coefficient of thermal expansion of the filter (1, 4) is smaller than 3 x 10⁻⁶/°C (at room temperature).

4. Laser protective goggles according to one of Claims 1-3, characterized in that, in the range of 20°C - 300°C, the linear coefficient of thermal expansion of the filter (1, 4) is smaller than 0.1 x 10⁻⁶/°C.

5. Laser protective goggles according to one of Claims 1-4, characterized in that the filter (1, 4) is constructed from two glass ceramic layers (2, 3, 5, 6).

6. Laser protective goggles according to one of Claims 1-5, characterized in that the inner, rear layer (6) of the filter (4) is designed as a correcting lens.

## Revendications

1. Lunettes protectrices contre les rayons laser, dans lesquelles on insère un filtre de protection (1,4) contre le rayonnement de lasers au CO₂, qui est constitué d'une ou plusieurs couches (2,3,5,6), au moins l'une des couches étant constituée du matériau vitrocéramique "Zerodur" (marque de la société Glaswerke Schott).

2. Lunettes protectrices contre les rayons laser selon la revendication 1, la couche du filtre (4) constituée de "Zerodur" étant agencée comme verre extérieur (5) devant les couches suivantes.

3. Lunettes protectrices contre les rayons laser selon l'une quelconque des revendications 1-2, caractérisées en ce que le coefficient de dilatation thermique linéaire (dans la plage de 20°C-300°C) du filtre (1,4) est inférieur à 3 x 10⁻⁶/°C (à température ambiante).

4. Lunettes protectrices contre les rayons laser selon l'une quelconque des revendications 1-3, caractérisées en ce que le coefficient de dilatation thermique linéaire (dans la plage de 20°C-300°C) du filtre (1,4) est inférieur à 0,1 x 10⁻⁶/°C.

5. Lunettes protectrices contre les rayons laser selon l'une quelconque des revendications 1-4,
caractérisées en ce que le filtre (1,4) est constitué de deux couches de vitrocéramique (2,3,5,6).

6. Lunettes protectrices contre les rayons laser selon l'une quelconque des revendications 1-5, caractérisées en ce que la couche arrière intérieure (6) du filtre (4) est conformée en verre correctif.
